# EUROPEAN PATENT SPECIFICATION

(11) **EP 3 747 506 B1**
(45) Date of publication and mention of the grant of the patent: **02.09.2026**
(21) Application number: 20160872.6
(22) Date of filing: 04.03.2020
(51) Int. Cl.: A61N 1/372, A61N 1/375

(54) **IMPLANTABLE PULSE GENERATOR WITH ANTENNA**
IMPLANTIERBARER IMPULSGENERATOR MIT ANTENNE
GÉNÉRATEUR D'IMPULSIONS IMPLANTABLE AVEC ANTENNE

(30) Priority: 05.06.2019 EP 19178331
(43) Date of publication of application: 09.12.2020
(73) Proprietor: ONWARD Medical N.V., 5611 ZX Eindhoven (NL)
(72) Inventor: Pflug, Hans, 5656 AE Eindhoven (NL); Tol, Jeroen, 5656 AE Eindhoven (NL); Koen, Weijand, 03580 l' Alfàs del Pi, Alicante (ES)
(74) Representative: WENDE IP GbR

(56) References cited:
- EP-A1- 3 269 423
- WO-A1-2015/069519
- US-A- 6 115 634
- US-A1- 2012 130 206

## Description

The present invention relates to an implantable medical device (IMD), preferably an implantable pulse generating system.

Pulse generating systems are known from various medical applications, inter alia from pacemakers and neuromodulation applications, such as neuromodulation for the treatment of a subject, e.g. in the field of improving recovery after neurological disorders such as spinal cord injury and/or stroke.

In particular, such pulse generation systems often are implantable pulse generating systems and are used in systems to deliver adaptive electrical spinal cord stimulation to facilitate and restore locomotion after neuromotor impairment as e.g. described in EP 2 868 343 A1.

US 7 813 809 B2 describes an implantable pulse generator (IPG) for prosthetic or therapeutic stimulation of muscles, nerves, or central nervous system tissue, or any combination, sized and configured to be implanted in subcutaneous tissue. The IPG includes a case and a control circuitry located within the case, and includes a primary cell or rechargeable power source, a receive coil for receiving an RF magnetic field to recharge the rechargeable power source, non-inductive wireless telemetry circuitry, and a microcontroller for control of the IPG.

Communication and power charging systems in a medical field are known from the prior art and especially used to submit energy and communication signals transcutaneously, in particular between a non-implanted device and an IPG.

For example, US 2015/0012061 A1 relates to a medical device for providing a stimulation therapy including a coil configured to receive both inductive charging and telemetry signals. The inductive charging signals are in a first frequency band. The telemetry signals are in a second frequency band higher than the first frequency band. The medical device includes inductive charging circuitry configured to provide electrical power to the medical device via the inductive charging signals. The medical device includes telemetry circuitry configured to conduct telecommunications with a external device via the telemetric signals. The medical device includes a first component electrically coupled between the coil and the inductive charging circuitry. The first component is configured to allow the inductive charging signals to pass through. The medical device includes a second component electrically coupled between the coil and the telemetric circuitry. The second component is configured to substantially block the inductive charging signals while allowing telemetric signals to pass through.

Alternative solutions are for example disclosed by EP 1 680 182 A1, EP 1 675 648 A1 and EP 1 575 665 A1.

Of note, IPG coils are usually attached to the exterior of the housing of the IPG or placed in the housing, in particular the main housing. The first approach results in a larger size of the IPG in terms of its largest width and thus is an adverse option, as the larger the size of the IPG is, the worse it is for the patient. The second approach is also an adverse option due to the conducting properties of the usually used titanium housing. A conductive housing restricts the usable frequency band of inductive charging and communication signals, and consequently, the charging performance, e.g. maximum IPG charging current and charging distance to a non-implantable charging device, and communication band options.

Preferably, the coil could be placed in an IPG header which is typically made of nonconducting material and attached to the IPG housing or IPG main housing. This represents one of the most practical solutions of integrating a coil into an IPG, both in terms of form factor of the IPG and in terms of IPG charging and communication performance.

However, as far as charging is concerned, this introduces the risk of implanting the IPG with the side of the header containing the coil not closest to the skin of a patient if a common a-symmetric header coil design is used, effectively increasing the implantation depth of the IPG header coil. This may adversely increase the implantation depth of the header coil by half the header thickness and thus may reduce the performance of the wireless charging link. In other words, the distance between the IPG (header) coil and the coil of the related non-implanted device sending signals to the IPG is not optimal.

It is an object of the present invention to improve an antenna and/or an induction coil of an IPG of a neuromodulation and/or neurostimulation system, in particular in that a better performance of the IPG in terms of increasing a coupling coefficient or coupling factor of an inductive wireless link and therefore the neuromodulation and/or neurostimulation system is enabled.

This object is solved according to the present invention with an implantable medical device with the features of claim 1.

The invention is based on the basic idea that, by designing an antenna for an IMD, e.g. an IPG, e.g. an IPG for neuromodulation and/or neurostimulation, comprising at least one coil or one set of coils, each coil comprising several windings, one may avoid that the distance between coil and skin of a patient implanted with the IMD is not minimal. Thus, it does not matter which side of the IMD, e.g. which side of an IPG, faces the skin of the patient and thus is closer to the related non-implanted device. In other words, the features of the antenna according to the present invention may enable placing the antenna in minimal distance to the skin of the patient, independent of its orientation, thus in minimal distance for inductive wireless power transfer and/or near field magnetic induction (NFMI) communication. The antenna design is therefore optimized for transferring a required amount of power from a non-implanted device to the IMD, in particular to an IMD battery charging circuit to charge the battery. Further, the antenna design may be optimal for communication between a non-implanted device and the IMD (bidirectionally, using NFMI). Overall, the antenna design may enable better charging and/or communication performance of the IMD and optimized neuromodulation/ neurostimulation.

In general, the IMD may be an active medical device, a pacemaker, a monitoring device, a drug administration device or an IPG, in particular a cochlear implant, a diaphragm stimulator, a sphincter stimulator, a bladder stimulator, and/or a neurostimulator and/or neuromodulator.

A set of coils may be or may comprise at least two coils. However, any number of coils more than two may be generally possible.

Each coil may be at least partially made of wire, in particular made of gold wire. A coil made of gold wire may have the advantage that it is relatively unsusceptible to corrosion.

In particular, the wire may have a diameter of 0.05mm to 0.5mm. The thickness of the wire may be adjusted to the self-guiding behavior of the wire. Due to the self-guiding behavior of the wire, it may be possible that the wire guide can also run continuously during orthocyclic winding and does not need to follow step by step.

In particular, the inductive wireless power transfer may be a resonant inductive wireless power transfer.

Further, the at least one coil or the coils of the at least one set of coils each may form a rectangular form, especially a trapezoidal form. Beyond the number of windings, the inductance of a coil also results from the dimension of a coil. Given that the housing of IMDs, e.g. IPGs, or certain parts of IMDs, e.g. certain parts of IPGs, such as a header, are often characterized by a rectangular form, especially a trapezoidal form, the coil or the coils of the at least one set of coils forming a rectangular form, especially a trapezoidal form, may enable maximum use of the dimension of the housing of the IMD, e.g. of the IPG. Thus, using coils each forming a rectangular form, especially a trapezoidal form, may enable a maximum level of inductance relative to the given dimensions of the housing of the IMD, e.g. of the IPG.

Further, the coils of the at least one set of coils may be connected by means of at least one bridging element. This connection between the coils of the at least one set of coils may enable that the inductance of a first coil may be combined with the inductance of a second coil. In other words, the current flowing in one coil may induce a voltage in the adjacent, second coil, finally causing an increased mutual inductance of the coils of the at least one set of coils.

According to the present invention, an IMD comprising at least one antenna as described above is disclosed, especially an IPG comprising at least one antenna as described above is disclosed.

In particular, the IMD may have a main housing part and a header part attached to the main housing part, wherein the at least one antenna may be arranged in the header part. As mentioned above, this arrangement may result in the most practical solution of integrating a coil into an IMD, in terms of form factor of the IMD, available assembly space and in terms of performance of the IMD.

An IMD antenna and/or coil may be used for 5-8 MHz, especially 6.78 MHz, inductive wireless power transfer, e.g. resonant inductive wireless power transfer, and 8-12 MHz, especially 10.6 MHz, NFMI communication. Arranging the antenna in the metal housing of an IMD, such as titanium, may be disadvantageous when using the antenna and/or coil for 5-8 MHz, especially 6.78 MHz, inductive wireless power transfer, e.g. resonant inductive wireless power transfer, and 8-12 MHz, especially 10.6 MHz, near field magnetic induction communication, due to the conducting properties of the housing leading to eddy-current losses and severe signal attenuation. Thus, arranging the IMD antenna and/or coil inside the titanium housing part of the IMD may be not desirable. Further, arranging the antenna around the housing of the IMD may be disadvantageous and not desirable as this may result in a larger size of the IMD in terms of its largest width while at the same time adding cumbersome manufacturing steps to the IMD production. Arranging the antenna in the header part may overcome these disadvantages.

Further, the header part may be transparent. By using a transparent header part, it may be enabled that the integrity of the coils may be checked visually at the end of IMD production, e.g. for quality control reasons.

In particular, the header part may be at least partially made of a polymer such as but not limited to epoxy, polyurethane and/or silicone. In particular, the header part may be made of medical grade material. In particular, the header may be at least partially made of a medical grade polymer such as but not limited to medical grade epoxy, medical grade polyurethane and/or medical grade silicone. Alternatively, and/or additionally, the header part may be made of medical grade polyamide and/or medical grade polypropylene. As the header may be made of non-conductive material it may not support or function as an indifferent electrode due to eddy-current losses, leading to better performance of the IMD.

Further, the header at least partially made of a polymer such as but not limited to epoxy, polyurethane and/or silicone may have insulation effects.

The coils of the at least one set of coils may be arranged in parallel (in a geometrical sense - electrically arranged in series or may also be arranged electrically in parallel). In particular, arranging a first coil comprising several windings and a second coil comprising several windings in parallel may enable maximal total (mutual) inductance. The geometrical parallel arrangement may be beneficial for the charging function.

Alternatively, the coils of the at least one set of coils each comprising several windings may be arranged orthogonally to each other (in a geometrical sense).

Alternatively, the sets of windings can be arranged orthogonally to each other.

Further, the antenna may comprise at least one further coil or at least one further set of coils. In particular, the at least one further coil or at least one further set of coils may increase the coupling factor between the antenna of the implanted IMD and an antenna and/or coil of a non-implanted device which needs to charge the IMD battery or communicate with the IMD for controlling reasons.

In particular, the antenna may comprise at least one further coil or at least one further set of coils, wherein the at least one further coil or one further set of coils may be arranged substantially orthogonally with regard to the at least one set of coils, i.e. the first set of coils.

During communication, the non-implanted device may be in a non-optimal orientation with regard to the at least one coil or the coils of the at least one set of coils each comprising several windings arranged in parallel geometrically, resulting in a low coupling factor, e.g. coupling factor null. By introducing at least one further coil or at least one further set of coils comprising several windings, especially substantially orthogonally with regard to the at least one coil or at least one set of coils each comprising several windings, e.g. in an IPG header, communication quality and performance may be improved for these non-optimal orientations without impacting the charging performance much.

Alternatively, the at least one further coil or the at least one further set of coils may be arranged substantially parallelly with regard to the at least one set of coils.

Alternatively, the at least one further coil or the at least one further set of coils may be arranged substantially at an acute or obtuse angle with regard to the at least one set of coils.

If windings are geometrically arranged rectangularly or substantially rectangularly, this can be used to the so-called nulls in case of NFMI use.

Further, the header part of the IMD, e.g. IPG, may comprise a mechanical support structure comprising at least one channel structure in which each winding of a coil is received and wound around the support structure. The mechanical support structure may enable forming and keeping the form of the at least one coil or the coils of the at least one set of coils, such as a rectangular, especially a trapezoidal form. The mechanical support structure may additionally and/or alternatively enable keeping one coil apart from another coil.

The IMD, e.g. the IPG, comprises connector slots being arranged at least partially within the at least one antenna.

Generally, an IMD header, e.g. an IPG header, may include one or more connector-slots and housing feed-throughs, typically located in the center of the housing-side. Mounting the connectors in the header center may make the header construction easier. According to the present invention, this construction advantage is maintained with the connector slots being arranged at least partially within the at least one antenna.

Further, the connection of the connector slots with the housing-feedthroughs may be more cumbersome in case the connectors are placed more towards one side of the header (outside of its center). This may require bending of the connector connections towards the feedthroughs, therefore requiring more header height and/or width. Therefore, placing the connectors at least partially within the at least one antenna, e.g. in the middle of the antenna, and having the antenna next to the connectors on both sides may lead to a lower volume claim of the antenna in the header and/or the header itself.

Further, the IMD may be or may comprise a switch interface which may decide which of the coils and/or set of coils to use, wherein each coil and/or set of coils comprises its own feed-through and/or is connected to at least one feed-through assigned to the coil. This means that each coil has its own feed-through. This feet-through can be only assigned to this coil and directly connected to it. It is possible that this feed-through is not connected to another coil (directly). In particular, electronics inside the IMD may enable to decide which coil and/or set of coils to use, for example based on a received signal strength (e.g. NFMI signal strength) or charge current strength (charging current magnitude). In particular, this may increase the degree of freedom on electronic functionality, as the antenna configuration may be changed according to the situation.

Further details and advantages of the present invention shall now be disclosed in connection with the drawings.

It is shown in:
- Fig. 1: a schematic perspective view of a first embodiment of an antenna together with an implantable pulse generator according to the present invention;
- Fig. 2a: a schematic top view of the first embodiment of the antenna together with the implantable pulse generator according to Fig. 1 in more/greater detail;
- Fig. 2b: a schematic side view of the first embodiment of the antenna together with the implantable pulse generator according to Fig. 1 in more/greater detail;
- Fig. 3a: a diagram of a coil coupling coefficient between the implanted IPG coil and the non-implanted device coil of the implanted antenna of the implantable pulse generator according to Fig. 1;
- Fig. 3b: a further diagram of a coil coupling coefficient between the implanted IPG coil and the non-implanted device coil of the implanted antenna of the implantable pulse generator according to Fig. 1; and
- Fig. 4: a schematic view of a mechanical support structure comprised in the header part of the IPG disclosed in Fig. 1.

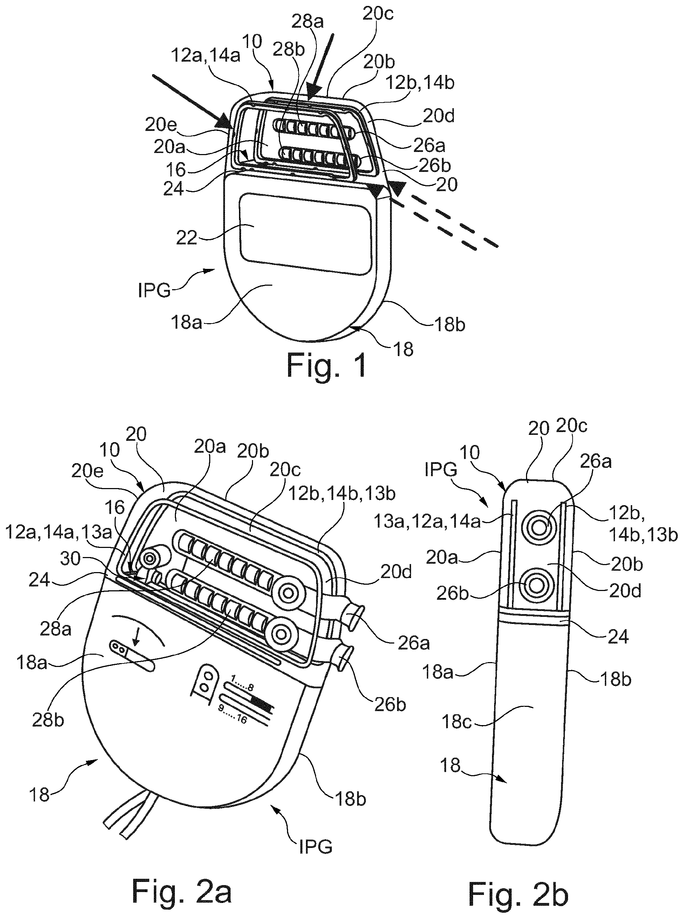

**Fig. 1** shows a schematic perspective view of a first embodiment of an antenna 10 together with an implantable pulse generator IPG according to the present invention.

In this embodiment, an antenna for an implantable medical device IMD is shown.

The antenna 10 comprises a set of coils 12a, 12b, wherein each coil 12a, 12b comprises several windings 14a, 14b, respectively.

In other words, the antenna 10 comprises two coils 12a, 12b.

In this embodiment, the coils 12a, 12b comprise an identical number of windings 14a, 14b.

The number of windings 14a, 14b of each coil 12a, 12b is four.

According to a further embodiment, the number of windings 14a, 14b of each coil 12a, 12b is an integer being bigger or smaller than four.

Generally, the number of windings 14a, 14b may be chosen from the range 1-10 windings 14a, 14b.

In one alternative embodiment, the number of windings 14a, 14b of each coil 12a, 12b is two.

The coils 12a, 12b form an induction-coil having an overall number of eight windings 14a, 14b (or so-called turns).

Thus, the coils 12a, 12b are electrically connected by means of a bridging element 16.

In general, the connection between the coils 12a, 12b is not necessarily an electric connection.

In other words, the coils 12a, 12b could be connected by means of a bridging element 16.

In an alternative embodiment, the coils 12a, 12b could be connected by means of more than one bridging element 16.

Each of the two coils 12a, 12b form a rectangular form.

Especially in the shown embodiment, each of the two coils 12a, 12b form a trapezoidal form.

As shown, the windings 14a, 14b are arranged in term of geometry in parallel.

As shown, the coils 12a, 12b of the at least one set of coils 12a, 12b are arranged in parallel.

Further, **Fig. 1** shows an implantable pulse generator IPG comprising the antenna 10 as mentioned above.

In other words, in this embodiment, the implantable medical device IMD is an implantable pulse generator IPG.

The implantable pulse generator IPG has a main housing part 18.

The main housing part 18 is formed of a medical-grade metal alloy such as titanium alloys Ti-6Al-4V-ELI or TAV-ELI.

The main housing part 18 constitutes a thin-walled and shell-like housing structure which surrounds the inner components of the implantable pulse generator IPG in a fluid tight sealed manner.

Most important inner components are the battery 22 and the control electronics (not shown in **Fig. 1****)** coupled thereto for generating specific stimulation patterns for neuromodulation, especially neurostimulation, after spinal cord injuries and/or stroke as known in the prior art.

On the top face (based on an implanted condition) of the main housing part 18, which is arranged opposite to its bottom, a header part 20 is attached to the main housing part 18.

According to **Fig. 1****,** the antenna 10 is arranged in the header part 20.

At the top face of the main housing part 18, this main housing part 18 and the header part 20 form, at a corresponding sealing face, a further fluid tight sealing with an elastomeric O-Ring 24 having a rectangular shape.

The header part 20 has a substantially trapezoidal form.

The header part 20 consists of a first and second main surface 20a, 20b (each with the biggest surface area of the header part 20) arranged in parallel.

Further, the first and second main surface 20a, 20b flush with the two planar outer surfaces 18a, 18b of the main housing part 18 being also arranged in parallel with regard to each other.

The header part 20 further comprises one header part top surface 20c being orientated opposite of the joint sealing face formed by the main housing part 18 and the header part 20.

The header part 20 also comprises two side surfaces 20d, 20e extending between the sealing face and the header part top surface 20c on the one hand.

On the other hand, these two side surfaces 20d, 20e also extend between the two main surfaces 20a, 20b.

The two coils 12a, 12b are arranged at the first and second main surfaces 20a, 20b of the header part 20.

Consequently, the two coils 12a, 12b form a symmetrical arrangement within the header part 20 (as indicated by the two parallel, dashed arrows).

Especially, the two coils 12a, 12b form a parallel arrangement within the header part 20.

Not shown in **Fig. 1** is that there could be at least one further coil 12 and/or at least one further set of coils 12.

Not shown in **Fig. 1** is that there could be a third coil 12 and/or a fourth coil 12.

The two further arrows of **Fig. 1** (continuous lines) indicate further optional locations of an additional third and/or fourth coil 12.

Not explicitly shown in **Fig. 1** is that the third coil 12 could be arranged at one side surface 20d, 20e.

Alternatively, the third coil 12 could be arranged at both side surfaces 20d, 20e.

Additionally, or alternatively, the fourth coil 12 could be arranged at the header part top surface 20c.

These additional third and fourth coils 12 may each have a substantially orthogonal orientation with respect to the first and second coil 12a, 12b.

Alternatively, the third or fourth coil 12 may have a substantially orthogonal orientation with respect to the first or the second coil 12a, 12b.

In other words, the antenna could generally comprise at least one further coil 12, wherein the at least one further coil 12 could be arranged substantially orthogonally with regard to the at least one set of coils 12a, 12b.

Not shown in **Fig. 1** is that the third and/or fourth coil 12 is/are built up in the same manner as the first and second coil 12a, 12b.

Generally possible is the use of a different number of windings/turns 14, 14a, 14b for each coil 12, 12a, 12b, for example, to implement a weighing factor between the different orthogonal orientations.

Alternatively, the at least one further coil 12 or the at least one further set of coils 12 could be arranged substantially parallelly with regard to the at least one set of coils 12.

Alternatively, the at least one further coil 12 or the at least one further set of coils 12 could be arranged substantially at an acute or obtuse angle with regard to the at least one set of coils 12.

Generally, the at least one winding 14 of a further coil 12 could be referred to as at least one additional winding 14.

This additional at least one winding14 improves the coil 12, 12a, 12b coupling between the non-implanted external device (not shown in **Fig. 1****)** and the implantable pulse generator IPG in case of a non-optimal orientation between the external device and the implantable pulse generator IPG, especially for NFMI communication.

As can be further depicted from **Fig. 1****,** the header part 20 is transparent.

Not directly shown **Fig. 1** is that the header part 20 is at least partially made of polymer.

In this embodiment, the header part 20 is fully made of polymer.

In this embodiment, the polymer is epoxy.

In an alternative embodiment, the polymer may be but not limited to epoxy, polyurethane and/or silicone.

In general, the polymer may be but not limited to a medical grade epoxy, medical grade polyurethane and/or medical grade silicone.

Alternatively, and/or additionally, the header part may be made of medical grade plastic such as medical grade polyamide or medical grade polypropylene.

Not shown in **Fig. 1** is that the coils 12, 12a, 12b are made of wire.

Not shown in **Fig. 1** is that the coils 12, 12a, 12b are made of gold wire.

Generally, each coil 12, 12a, 12b can be at least partially made of wire, in particular gold wire.

Not shown in **Fig. 1** is that the wire has a has a diameter of 0.05mm to 0.5mm.

Not shown in **Fig. 1** is that the implantable pulse generator IPG could in an alternative embodiment be any other type of implantable medical device IMD.

Not shown in **Fig. 1** is that the IMD, in particular the IPG, comprises an (electronic) switch interface which decides which of the coils 12, 12a, 12b and/or set of coils 12, 12a, 12b to use, wherein each coil 12, 12a, 12b and/or set of coils 12, 12a, 12b comprises its own feed-through and/or is connected to at least one feed-through assigned to the coil.

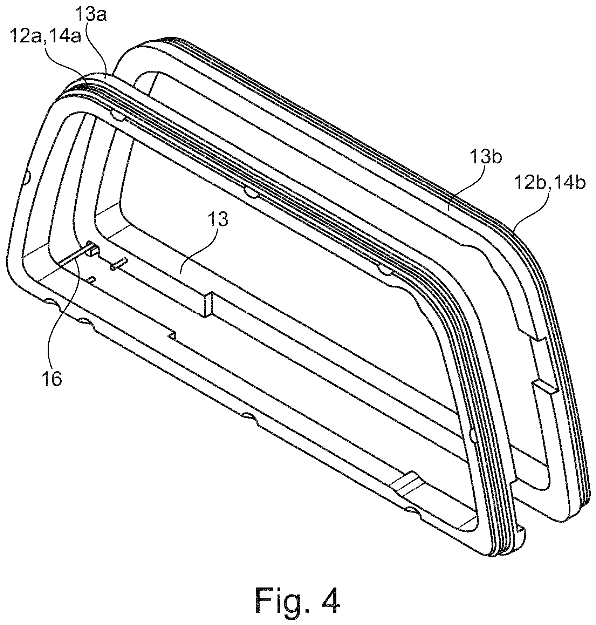

Not shown in **Fig. 1** is that the header part 20 (also) comprises a mechanical support structure 13, cf. **Fig. 4****.**

The mechanical support structure 13 has a rectangular form, in particular a trapezoidal form.

The mechanical support structure 13 is made of non-conductive material.

In this embodiment, the mechanical support structure 13 is made of plastic.

In this embodiment, the mechanical support structure 13 comprises two channel structures 13a, 13b.

In this embodiment, the two channel structures 13a, 13b are separated from each other.

The mechanical support structure 13 is carrying the two coils 12a, 12b.

In particular, the channel structure 13a is carrying coil 12a.

In particular, the channel structure 13b is carrying coil 12b.

In other words, each channel structure 13a, 13b is configured to carry one coil 12a, 12b.

The mechanical support structure 13 is carrying the windings 14a, 14b.

In particular, the channel structure 13a is carrying the windings 14a.

In particular, the channel structure 13b is carrying the windings 14b.

In general, the mechanical support structure 13 forms the form of the coils 12a, 12b.

In general, the header part 20 comprises a mechanical support structure 13 comprising at least one channel structure 13a, 13b in which each winding 14a, 14b of a coil 12a, 12b is received and wound around the support structure 13.

**Fig. 2a** shows a schematic top view of the first embodiment of the antenna 10 together with the implantable pulse generator IPG according to **Fig. 1** in more detail.

Especially, the two coils 12a, 12b are arranged at the edges of the first and second main surfaces 20a, 20b of the header part 20.

For example, the two coils 12a, 12b of the windings 14a, 14b are arranged at the edges of the first and second main surfaces 20a, 20b of the header part 20 by embedding them therein.

The embedding can be done as here for example shown by overmolding.

Alternatively, and/or additionally, injection molding is also possible.

Further, the optional third and/or fourth coils is/are integrated in the side and header part top surface(s) 20c, 20d, 20e in the same manner as described for the main surfaces 20a, 20b.

As can be depicted in greater detail in **Fig. 2a****,** the two coils 12a, 12b are connected by a bridging element 16.

The bridging element 16 may be optionally also used for further connection of the third and/or fourth coil 12.

In an alternative embodiment (not shown), all coils 12, 12a, 12b may be equipped with their own feed-through connections and these coils may be electronically connected, i.e. via a connection matrix, in a desired configuration depending on e.g. received charge current or received NFMI signal strength.

This bridging element 16 is also embedded within the header part 20 and extends between the two coils 12a, 12b at one or both side surfaces 20d, 20e.

As can be further depicted from **Fig. 2a****,** the implantable pulse generator IPG, at its header part 20 comprises a first and second connector slot 26a, 26b for connecting the two corresponding lead connectors 28a, 28b with the two control leads of the spinal cord stimulation paddle (not shown in **Fig. 2a****).**

These connector slots 26a, 26b are arranged partially or completely within the antenna 10, i.e. within the projected area formed by the first and second coils 12a, 12b of.

Additionally, the two coils 12a, 12b, the connector slots 26a, 26b, and the corresponding lead connectors 28a, 28b form a sandwich-like structure with the connector slots 26a, 26b and the corresponding lead connectors 28a, 28b disposed in between the two coils 12a, 12b.

Especially, the connector slots 26a, 26b are arranged in one of the side surfaces 20d, 20e of the header part 20.

This arrangement causes the third coil 12 to be arranged at the opposite side surface 20d, 20e of the side surface 20d, 20e with the connector slots 26a, 26b disposed therein.

The two coils 12a, 12b (or optionally the third and/or fourth coils 12) are further connected with the control electronics and the battery 22 (each not shown in Fig. 2a) inside the main housing part 18 via a connection area 30 with feed-throughs serving as a connection interface.

Furthermore, a plurality of connecting wires connecting each terminal of the two lead connectors 28a, 28b to the corresponding pins of the feed-through capacitors of control electronics are also shown in greater detail in **Fig. 2a****.**

**Fig. 2b** shows a schematic side view of the first embodiment of the antenna 10 together with the implantable pulse generator IPG according to Fig. 1 in more detail.

Especially, the individual windings 14a, 14b of the first and second coil 12a, 12b may be depicted in more detail.

Additionally, a weld 18c in the center of the main housing part 18 is visible therein as the main housing part 18 is built up by welding two housing part shells together.

Additionally, the function of the antenna 10 as shown in **Figs. 1** to **Fig. 2b** together with the implantable pulse generator IPG is as follows:
The antenna 10 for an implantable pulse generator IPG is configured for inductive wireless power transfer and/or near field magnetic induction (NFMI) communication.

Not shown in **Fig. 2b** is that the inductive wireless power transfer could generally be resonant inductive wireless power transfer.

The antenna 10 for an implantable pulse generator IPG is configured for 6.78 MHz inductive wireless power transfer and for 10.6 MHz near field magnetic induction (NFMI) communication.

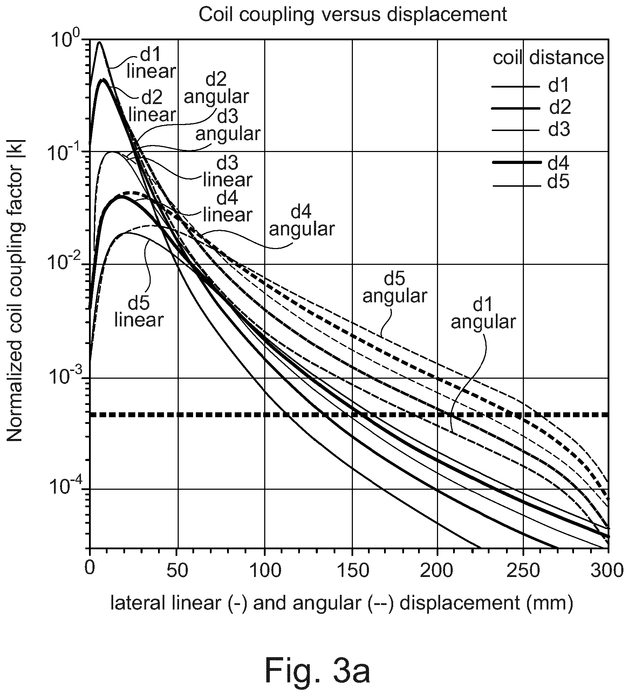

In this context, **Fig. 3a** shows a diagram of a coil coupling coefficient or factor k between the implanted IPG coil 12a,b and the non-implanted device coil of the implanted antenna 10 of the implantable pulse generator IPG according to **Fig. 1** to **Fig. 2b****.**

The coil coupling coefficient k is shown between an external and non-implanted coil (e.g. a charging or communication coil) and the implanted antenna 10 of the implantable pulse generator IPG versus a lateral displacement (both linear and in an angular fashion around a round body model) of the antenna 10.

This graph corresponds to a communication use-case, showing (as a dashed horizontal line) the coupling factor k corresponding to the applied receiver sensitivity level, assuming a specific known transmitter output level.

In **Fig. 3a****,** the coupling factor k of the antenna 10 is shown without additional orthogonal coils 12 (e.g. at the side surface 20d, 20e and/or header top surface 20c).

The non-implanted coil for communication is a different one, compared to the used wireless charging coil but one could also use a single coil for both modalities on the external device side.

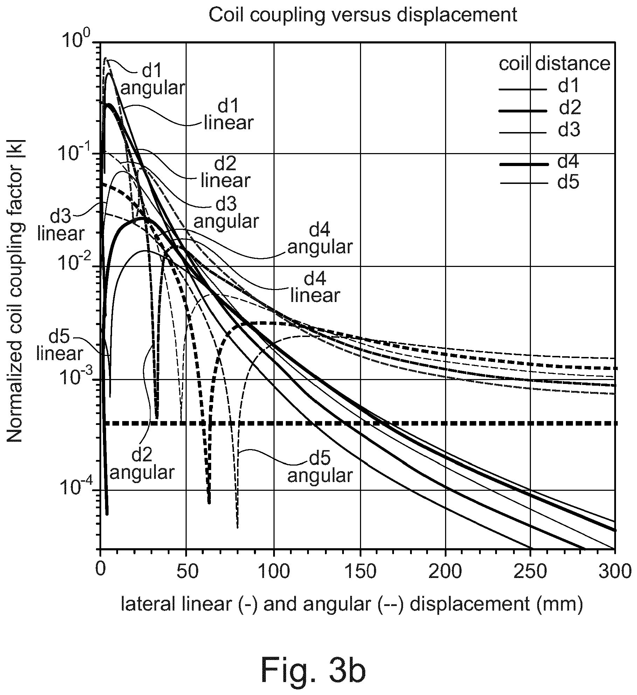

**Fig. 3b** shows, in contrast to **Fig. 3a****,** a further diagram of a coil coupling coefficient k between the implanted IPG coil and the non-implanted device coil of the implanted antenna of the implantable pulse generator according to **Fig. 1** to **Fig. 2b****.**

Compared to the set up as described in **Fig. 3a****,** the following modifications of the antenna 10 have been implemented:
In contrast to **Fig. 3a****,** **Fig. 3b** shows the coupling factor k of the antenna 10 with at least one additional orthogonal coil 12 (see **Fig. 1****,** e.g. at the side surface 20d, 20e and/or header top surface 20c), showing an improvement in the k-factor, especially for the angular case (dashed line), leading to a larger communication range.

Further, it turns out that the charging performance is not meaningfully impacted by the added orthogonal set(s) of windings in the header 20.

Thus, the header antenna 10 (coils12a, 12b at the main surfaces 20a, 20b) may still be used for both charging and communication.

**Fig. 4** shows a schematic view of a mechanical support structure 13 comprised in the header part of the IPG disclosed in **Fig. 1****.**

In particular, a mechanical support structure 13 comprising two channel structures 13a, 13b in which each winding 14a, 14b of the two coils 12a, 12b, of the antenna 10 disclosed in Fig. 1 is received and wound around the support structure is shown.

The coils 12a, 12b are connected by means of a bridging element 16.

### References

- 10: antenna
- 12: coil
- 12a: coil
- 12b: coil
- 13: mechanical support structure
- 13a: channel structure
- 13b: channel structure
- 14: windings
- 14a: windings
- 14b: windings
- 16: bridging element
- 18: main housing part
- 18a: planar outer surface
- 18b: planar outer surface
- 18c: weld
- 20: header part
- 20a: first main surface
- 20b: second main surface
- 20c: header part top surface
- 20d: side surface
- 20e: side surface
- 22: battery
- 24: O-Ring
- 26a: connector slot
- 26b: connector slot
- 28a: lead connector
- 28b: lead connector
- 30: connection area

- IMD: implantable medical device
- IPG: implantable pulse generator

## Claims

1. An implantable medical device (IMD) comprising at least one antenna (10),
wherein the antenna (10) is configured for inductive wireless power transfer and/or near-field magnetic induction communication, the antenna (10) comprising at least one set of coils (12a, 12b), each coil (12a, 12b) comprising several windings (14a, 14b),
wherein the implantable medical device (IMD) comprises connector slots 26a, 26b, **characterized in that** the connector slots are arranged at least partially within the at least one antenna.

2. The implantable medical device (IMD) according to claim 1, **characterized in that** the inductive wireless power transfer is a resonant inductive wireless power transfer.

3. The implantable medical device (IMD) according to claim 1 or claim 2, **characterized in that** each coil (12a, 12b) is at least partially made of wire, in particular made of gold wire.

4. The implantable medical device (IMD) according to one of the preceding claims, **characterized in that** the wire has a diameter of 0.05mm to 0.5mm.

5. The implantable medical device (IMD) according to one of the preceding claims, **characterized in that** the coils (12a, 12b) of the at least one set of coils (12a, 12b) comprise an identical number of windings (14a, 14b).

6. The implantable medical device (IMD) according to one of the preceding claims, **characterized in that** the number of windings (14a, 14b) is chosen from the range 1-10 windings (14a, 14b).

7. The implantable medical device (IMD) according to one of the preceding claims, **characterized in that** the coils (12a, 12b) of the at least one set of coils (12a, 12b) each form a rectangular form, especially a trapezoidal form.

8. The implantable medical device (IMD) according to one of the preceding claims, **characterized in that** the coils (12a, 12b) of the at least one set of coils (12a, 12b) are connected by means of a least one bridging element (16).

9. The implantable medical device (IMD) according to one of the preceding claims, wherein the implantable medical device (IMD) is an implantable pulse generator (IPG).

10. The implantable medical device (IMD) according to one of the preceding claims, **characterized in that** the implantable medical device (IMD) has a main housing part (18) and a header part (20) attached to the main housing part (18), wherein the at least one antenna (10) is arranged in the header part (20).

11. The implantable medical device (IMD) according to one of claim 10, **characterized in that** the header part (20) is transparent.

12. The implantable medical device (IMD) according to one of the preceding claims, **characterized in that** the header part (20) is at least partially made of polymer such as but not limited to epoxy, polyurethane and/or silicone.

13. The implantable medical device (IMD) according to one of the preceding claims, **characterized in that** the coils (12a, 12b) of the at least one set of coils (12a, 12b) are arranged in parallel.

14. The implantable medical device (IMD) according to one of the preceding claims, **characterized in that** the antenna (10) comprises at least one further coil (12) or at least one further set of coils (12).

15. The implantable medical device (IMD) according to claim 14, **characterized in that** the at least one further coil (12) or the at least one further set of coils (12) is arranged substantially orthogonally with regard to the at least one set of coils (12a, 12b).

16. The implantable medical device (IMD) according to claim 14, **characterized in that** the at least one further coil (12) or the at least one further set of coils (12) is arranged substantially parallelly with regard to the at least one set of coils (12a, 12b).

17. The implantable medical device (IMD) according to claim 14, **characterized in that** the at least one further coil (12) or the at least one further set of coils (12) is arranged substantially at an acute or obtuse angle with regard to the at least one set of coils (12a, 12b).

18. The implantable medical device (IMD) according to one of the preceding claims, **characterized in that** the header part (20) comprises a mechanical support structure (13) comprising at least one channel structure (13a, 13b) in which each winding (14a, 14b) of a coil (12a, 12b) is received and wound around the support structure (13).

19. The implantable medical device (IMD) according to one of the preceding claims **characterized in that** the implantable medical device (IMD) comprises a switch interface which is configured and arranged to decide which of the coils (12, 12a, 12b) and/or set of coils (12, 12a, 12b) to use, wherein each coil (12, 12a, 12b) and/or set of coils (12, 12a, 12b) comprises its own feed-through and/or is connected to at least one feed-through assigned to the coil.

## Patentansprüche

1. Implantierbare medizinische Vorrichtung (IMD), umfassend mindestens eine Antenne (10),
wobei die Antenne (10) für eine induktive drahtlose Leistungsübertragung und/oder eine magnetische Nahfeldinduktionskommunikation konfiguriert ist, die Antenne (10) umfassend mindestens einen Satz von Spulen (12a, 12b), jede Spule (12a, 12b) umfassend mehrere Windungen (14a, 14b),
wobei die implantierbare medizinische Vorrichtung (IMD) Verbinderschlitze 26a, 26b umfasst,
**dadurch gekennzeichnet, dass** die Verbinderschlitze mindestens teilweise innerhalb der mindestens einen Antenne angeordnet sind.

2. Implantierbare medizinische Vorrichtung (IMD) nach Anspruch 1, **dadurch gekennzeichnet, dass** die induktive drahtlose Leistungsübertragung eine resonante induktive drahtlose Leistungsübertragung ist.

3. Implantierbare medizinische Vorrichtung (IMD) nach Anspruch 1 oder 2, **dadurch gekennzeichnet, dass** jede Spule (12a, 12b) mindestens teilweise aus Draht hergestellt ist, insbesondere aus Golddraht hergestellt ist.

4. Implantierbare medizinische Vorrichtung (IMD) nach einem der vorstehenden Ansprüche, **dadurch gekennzeichnet, dass** der Draht einen Durchmesser von 0,05 mm bis 0,5 mm aufweist.

5. Implantierbare medizinische Vorrichtung (IMD) nach einem der vorstehenden Ansprüche, **dadurch gekennzeichnet, dass** die Spulen (12a, 12b) des mindestens einen Satzes von Spulen (12a, 12b) eine identische Anzahl von Windungen (14a, 14b) umfassen.

6. Implantierbare medizinische Vorrichtung (IMD) nach einem der vorstehenden Ansprüche, **dadurch gekennzeichnet, dass** die Anzahl der Windungen (14a, 14b) aus dem Bereich von 1-10 Windungen (14a, 14b) ausgewählt ist.

7. Implantierbare medizinische Vorrichtung (IMD) nach einem der vorstehenden Ansprüche, **dadurch gekennzeichnet, dass** die Spulen (12a, 12b) des mindestens einen Satzes von Spulen (12a, 12b) jeweils eine rechteckige Form, insbesondere eine Trapezform, ausbilden.

8. Implantierbare medizinische Vorrichtung (IMD) nach einem der vorstehenden Ansprüche, **dadurch gekennzeichnet, dass** die Spulen (12a, 12b) des mindestens einen Satzes von Spulen (12a, 12b) mittels mindestens eines Überbrückungselements (16) verbunden sind.

9. Implantierbare medizinische Vorrichtung (IMD) nach einem der vorstehenden Ansprüche, wobei die implantierbare medizinische Vorrichtung (IMD) ein implantierbarer Impulsgeber (IPG) ist.

10. Implantierbare medizinische Vorrichtung (IMD) nach einem der vorstehenden Ansprüche, **dadurch gekennzeichnet, dass** die implantierbare medizinische Vorrichtung (IMD) ein Hauptgehäuseteil (18) und ein Kopfteil (20) aufweist, das an dem Hauptgehäuseteil (18) angebracht ist, wobei die mindestens eine Antenne (10) in dem Kopfteil (20) angeordnet ist.

11. Implantierbare medizinische Vorrichtung (IMD) nach einem der Ansprüche 10, **dadurch gekennzeichnet, dass** das Kopfteil (20) transparent ist.

12. Implantierbare medizinische Vorrichtung (IMD) nach einem der vorstehenden Ansprüche, **dadurch gekennzeichnet, dass** das Kopfteil (20) mindestens teilweise aus Polymer hergestellt ist, wie, aber nicht beschränkt auf, Epoxid, Polyurethan und/oder Silikon.

13. Implantierbare medizinische Vorrichtung (IMD) nach einem der vorstehenden Ansprüche, **dadurch gekennzeichnet, dass** die Spulen (12a, 12b) des mindestens einen Satzes von Spulen (12a, 12b) parallel angeordnet sind.

14. Implantierbare medizinische Vorrichtung (IMD) nach einem der vorstehenden Ansprüche, **dadurch gekennzeichnet, dass** die Antenne (10) mindestens eine weitere Spule (12) oder mindestens einen weiteren Satz von Spulen (12) umfasst.

15. Implantierbare medizinische Vorrichtung (IMD) nach Anspruch 14, **dadurch gekennzeichnet, dass** die mindestens eine weitere Spule (12) oder der mindestens eine weitere Satz von Spulen (12) im Wesentlichen orthogonal in Bezug auf den mindestens einen Satz von Spulen (12a, 12b) angeordnet ist.

16. Implantierbare medizinische Vorrichtung (IMD) nach Anspruch 14, **dadurch gekennzeichnet, dass** die mindestens eine weitere Spule (12) oder der mindestens eine weitere Satz von Spulen (12) im Wesentlichen parallel in Bezug auf den mindestens einen Satz von Spulen (12a, 12b) angeordnet ist.

17. Implantierbare medizinische Vorrichtung (IMD) nach Anspruch 14, **dadurch gekennzeichnet, dass** die mindestens eine weitere Spule (12) oder der mindestens eine weitere Satz von Spulen (12) im Wesentlichen in einem spitzen oder stumpfen Winkel in Bezug auf den mindestens einen Satz von Spulen (12a, 12b) angeordnet ist.

18. Implantierbare medizinische Vorrichtung (IMD) nach einem der vorstehenden Ansprüche, **dadurch gekennzeichnet, dass** das Kopfteil (20) einen mechanischen Tragstruktur (13) umfasst, umfassend mindestens eine Kanalstruktur (13a, 13b), in der jede Windung (14a, 14b) einer Spule (12a, 12b) aufgenommen und um die Tragstruktur (13) herum gewunden ist.

19. Implantierbare medizinische Vorrichtung (IMD) nach einem der vorstehenden Ansprüche, **dadurch gekennzeichnet, dass** die implantierbare medizinische Vorrichtung (IMD) eine Schalterschnittstelle umfasst, die konfiguriert und angeordnet ist, um zu entscheiden, welche der Spulen (12, 12a, 12b) und/oder welcher Satz von Spulen (12, 12a, 12b) zu verwenden ist, wobei jede Spule (12, 12a, 12b) und/oder jeder Satz von Spulen (12, 12a, 12b) ihre/seine eigene Durchführung umfasst und/oder mit mindestens einer der Spule zugeordneten Durchführung verbunden ist.

## Revendications

1. Dispositif médical implantable (IMD) comprenant au moins une antenne (10),
dans lequel l'antenne (10) est configurée pour un transfert d'énergie sans fil inductif et/ou une communication par induction magnétique en champ proche, l'antenne (10) comprenant au moins un ensemble de bobines (12a, 12b), chaque bobine (12a, 12b) comprenant plusieurs enroulements (14a, 14b),
dans lequel le dispositif médical implantable (IMD) comprend des fentes de connecteur 26a, 26b,
**caractérisé en ce que** les fentes de connecteur sont agencées au moins partiellement au sein de l'au moins une antenne.

2. Dispositif médical implantable (IMD) selon la revendication 1, **caractérisé en ce que** le transfert d'énergie sans fil inductif est un transfert d'énergie sans fil inductif résonant.

3. Dispositif médical implantable (IMD) selon la revendication 1 ou la revendication 2, **caractérisé en ce que** chaque bobine (12a, 12b) est au moins partiellement constituée de fil, en particulier constituée de fil d'or.

4. Dispositif médical implantable (IMD) selon l'une des revendications précédentes, **caractérisé en ce que** le fil a un diamètre de 0,05 mm à 0,5 mm.

5. Dispositif médical implantable (IMD) selon l'une des revendications précédentes, **caractérisé en ce que** les bobines (12a, 12b) de l'au moins un ensemble de bobines (12a, 12b) comprennent un nombre identique d'enroulements (14a, 14b).

6. Dispositif médical implantable (IMD) selon l'une des revendications précédentes, **caractérisé en ce que** le nombre d'enroulements (14a, 14b) est choisi dans la plage de 1 à 10 enroulements (14a, 14b).

7. Dispositif médical implantable (IMD) selon l'une des revendications précédentes, **caractérisé en ce que** les bobines (12a, 12b) de l'au moins un ensemble de bobines (12a, 12b) forment chacune une forme rectangulaire, en particulier une forme trapézoïdale.

8. Dispositif médical implantable (IMD) selon l'une des revendications précédentes, **caractérisé en ce que** les bobines (12a, 12b) de l'au moins un ensemble de bobines (12a, 12b) sont reliées au moyen d'au moins un élément de pontage (16).

9. Dispositif médical implantable (IMD) selon l'une des revendications précédentes, dans lequel le dispositif médical implantable (IMD) est un générateur d'impulsions implantable (IPG).

10. Dispositif médical implantable (IMD) selon l'une des revendications précédentes, **caractérisé en ce que** le dispositif médical implantable (IMD) a une partie boîtier principal (18) et une partie tête (20) attachée à la partie boîtier principal (18), dans lequel l'au moins une antenne (10) est agencée dans la partie tête (20).

11. Dispositif médical implantable (IMD) selon l'une revendication 10, **caractérisé en ce que** la partie tête (20) est transparente.

12. Dispositif médical implantable (IMD) selon l'une des revendications précédentes, **caractérisé en ce que** la partie tête (20) est au moins partiellement constituée de polymère tel que, mais sans s'y limiter, de l'époxy, du polyuréthane et/ou du silicone.

13. Dispositif médical implantable (IMD) selon l'une des revendications précédentes, **caractérisé en ce que** les bobines (12a, 12b) de l'au moins un ensemble de bobines (12a, 12b) sont agencées en parallèle.

14. Dispositif médical implantable (IMD) selon l'une des revendications précédentes, **caractérisé en ce que** l'antenne (10) comprend au moins une autre bobine (12) ou au moins un autre ensemble de bobines (12).

15. Dispositif médical implantable (IMD) selon la revendication 14, **caractérisé en ce que** l'au moins une autre bobine (12) ou l'au moins un autre ensemble de bobines (12) est agencé sensiblement de manière orthogonale par rapport à l'au moins un ensemble de bobines (12a, 12b).

16. Dispositif médical implantable (IMD) selon la revendication 14, **caractérisé en ce que** l'au moins une autre bobine (12) ou l'au moins un autre ensemble de bobines (12) est agencé sensiblement de manière parallèle par rapport à l'au moins un ensemble de bobines (12a, 12b).

17. Dispositif médical implantable (IMD) selon la revendication 14, **caractérisé en ce que** l'au moins une autre bobine (12) ou l'au moins un autre ensemble de bobines (12) est agencé sensiblement à un angle aigu ou obtus par rapport à l'au moins un ensemble de bobines (12a, 12b).

18. Dispositif médical implantable (IMD) selon l'une des revendications précédentes, **caractérisé en ce que** la partie tête (20) comprend une structure de support mécanique (13) comprenant au moins une structure de canal (13a, 13b) dans laquelle chaque enroulement (14a, 14b) d'une bobine (12a, 12b) est reçu et enroulé autour de la une structure de support (13).

19. Dispositif médical implantable (IMD) selon l'une des revendications précédentes, **caractérisé en ce que** le dispositif médical implantable (IMD) comprend une interface de commutation qui est configurée et agencée pour décider laquelle des bobines (12, 12a, 12b) et/ou un ensemble de bobines (12, 12a, 12b) utiliser, dans lequel chaque bobine (12, 12a, 12b) et/ou ensemble de bobines (12, 12a, 12b) comprend sa propre traversée et/ou est reliée à au moins une traversée attribuée à la bobine.
